Europäisches Patentamt

European Patent Office

Office européen des brevets

(11) Publication number: **0 323 157**
**A2**

## EUROPEAN PATENT APPLICATION

(21) Application number: **88312216.0**

(22) Date of filing: **22.12.88**

(51) Int. Cl.4: **C07K 7/00 , A61K 37/02**

(30) Priority: **24.12.87 AU 6101/87**

(43) Date of publication of application:
**05.07.89 Bulletin 89/27**

(84) Designated Contracting States:
**AT BE CH DE ES FR GB GR IT LI LU NL SE**

(71) Applicant: **THE UNIVERSITY OF MELBOURNE**
**Grattan Street**
**Parkville Victoria 3052(AU)**

(72) Inventor: **Kemp, Bruce Ernest**
**20 Kellett Grove**
**Kew 3101 Victoria(AU)**
Inventor: **McPhee, Dale**
**14 Freeman Street**
**North Fitzroy 3065 Victoria(AU)**
Inventor: **Doherty, Richard Ralph**
**31 King Street**
**Balwyn 3103 Victoria(AU)**

(74) Representative: **Ford, Michael Frederick et al**
**MEWBURN ELLIS & CO. 2/3 Cursitor Street**
**London EC4A 1BQ(GB)**

(54) Antiviral compounds and methods.

(57) Compounds for use in the treatment or prophylaxis of Human Immunodeficiency Virus (HIV) infection, which upon contact with an HIV particle, are capable of preventing or disrupting interaction between the gp41 and gp120 envelope proteins of the said particle in the contact region between the said proteins.

EP 0 323 157 A2

## ANTIVIRAL COMPOUNDS AND METHODS

The present invention relates to compounds for use in the treatment of prophylaxis of viral infections, and in particular, Human Immunodeficiency Virus (HIV).

HIV contains two envelope glycoproteins gp120 and gp41 which are derived from a common precursor, gp160 (Barre-Sinoussi et al., 1983; Gallo et al., 1984; Popovic et al., 1984; Sarngadharan et al., 1984; Shaw et al., 1985; Levy et al., 1984; Robey et al., 1985; Alan et al., Science, 1985; Barin et al., 1985; Montagnier et al., 1983 and Veronese et al., 1985).

The larger glycoprotein gp120 is exterior whereas the gp41 has a transmembrane spanning region. There is now strong evidence that gp120 binds to the CD4 protein on the surface of T lymphocytes, macrophages and other cells (Dalgleish et al., 1984; Klatzman et al., 1984 and McDougal et al., 1986).

Monoclonal antibodies to CD4 prevent or limit the infectivity and replication of HIV in T-Cells (Popovic et al., 1984 and McDougal et al., 1985). The virus preferentially binds to the surface of CD4 positive cells and virus binding is inhibited by monoclonal antibody to CD4 (McDougal et al., 1985). Moreover, virus infected cells bind and fuse with uninfected CD4 positive T-cells, resulting in the formation of syncytia that can be inhibited by monoclonal antibody to CD4 (Dalgleish, et al., 1984). In addition radioimmunoprecipitation experiments on lysates of virus-exposed CD4 positive T-cells, the CD4 molecule and the gp120 coprecipitate as a complex (McDougal, et al., 1986a). Human cell lines that do not express the CD4 molecule are not infected but if they are transfected with the human CD4 gene and express CD4, they will bind virus and permit viral replication (McDougal et al., 1986b).

Following binding of gp120 to CD4, virus is thought to enter the cell by an envelope protein mediated fusion of the viral and target cell membranes. Expression of the virus envelope proteins on the surface of infected cells mediates fusion with uninfected cells giving rise to multinucleated giant cells or syncytia. (Dalgleish et al., 1984; Klatzman et al., 1984; McDougal et al., 1986a; Sodroski, et al., 1986 and Lifson et al., 1986).

Using deletion and insertion mutations Kowalski et al., (1987) have studied the role of the envelope proteins in forming syncytia in CD4 positive cells. Mutations have been identified that decrease binding of the envelope protein to the CD4 molecule, prevent a post-binding fusion reaction, disrupt the anchorage of the envelope glycoprotein in the membrane, the association of the two subunits of the envelope glycoprotein or post-translational proteolytic processing of the envelope precursor protein.

There is no disulfide bonds between gp41 and gp120 since reduction of disulfide bonds does not increase the release of gp120 from infected cells (McDougal et al., 1986b). Because a number of mutations affect the gp120-g41 association, Kowalski et al., (1987) have suggested that gp120 makes multiple contacts with the extracellular region of gp41 and that these contacts are responsible for the association of gp120 with gp41 once they are formed from the gp160 precursor. These authors suggest that gp120 acts as a bifunctional molecule associating with gp41 via sites in the amino terminal half and the CD4 receptor via the carboxyl terminal region. The interaction of the carboxyl terminal region of the gp120 with CD4 depends on higher orders of structure since denaturation of gp120 eliminates CD4 binding to the receptor (McDougal et al., 1986b). The mutation studies of Kowalski et al., (1987) indicate multiple conserved regions critical for CD4 binding interspersed with hypervariable regions. Together these observations suggest that the amino acid sequences of gp120 responsible for interaction with the CD4 molecule are discontinuous and depend on tertiary structure for association with it.

Many attempts have been made to devise strategies to treat and/or prevent HIV infection and its associated clinical manifestations.

The goal of an antiviral therapeutic strategy is to interfere with one of the vital steps in the virus' infection cycle. These may include the binding of the virus to the host receptor, fusion to the plasma membrane, reverse transcription, integration or a post translational step, release of the virus or fusion of an infected cell with an uninfected cell. Since AIDS patients may have high levels of circulating antibodies it is apparent that this alone does not provide immunity once infected. To date reverse transcriptase inhibitors such as AZT have provided encouraging results in terms of prolonging the lives of AIDS patients, however drugs of this nature are not without serious side effects.

We have now surprisingly identified compounds which are capable of inhibiting viral infection.

Accordingly, in one broad aspect of the present invention, compounds are provided which are capable of inhibiting viral infection. These compounds, which may be peptides of varying length and amino acid compositions or non-peptide compounds such as drug molecules, are characterized by the ability to disrupt the interaction(s) between viral envelope proteins and receptors expressed on the surface of target cells.

Disruption of the interaction(s) between viral envelope proteins prevents binding or association of the envelope proteins with cellular receptors, and thus blocks viral uptake into target cells bearing such receptors.

With reference to the AIDS (or HIV) virus, the present invention provides compounds for use in the treatment or prophylaxis of a Human Immunodeficiency Virus (HIV) infection, which upon contact with an HIV particle or infected cells, are capable of preventing or disrupting interaction between the gp41 and gp120 envelope proteins of the said particle or infected cell in the contact region between the said proteins.

Generally, the compounds of the invention prevent or disrupt the interaction between the gp41 and gp120 envelope proteins of HIV 1 or HIV 2 in the putitative contact regions defined by the following amino acid sequences:

<u>HIV 1</u>:

```
            571     574           579              585        589  591
gp41   ...W  G  I  K  Q  L  Q  A  R  I  L  A  V  E  R  Y  L  K  D  Q  Q...
            99      102           107              113        117
gp120  ...D  M  V  E  Q  M  H  E  D  I  I  S  L  W  D  Q  S  L  K  P  C...
```

<u>HIV 2</u>:

```
            571     574           579              585        589  591
gp41   ...W  G  T  K  N  L  Q  A  R  V  T  A  I  E  K  Y  L  Q  D  Q  A...
            78      81            86               92         96   98
gp120  ...T  V  T  E  Q  A  I  E  D  V  W  H  L  F  E  T  S  I  K  P  C...
```

Preferably, the compounds of the invention may be represented by compounds containing the following amino acid sequences:

H E D I I S L W D Q S L K P C V K L T P L

or

G I K Q L Q A R I L A V E R Y L K D Q Q

or parts thereof.

A compound within the scope of this invention may have the following peptide sequence:

H E D I I S L W D Q S L K.

The amino acids of the above peptides may be altered by substitution, addition or deletion, as long as antiviral activity is not lost.

Non-peptide compounds which disrupt the interaction between HIV envelope proteins gp41 and gp120 are also within the scope of the present invention.

Because of the presence of hydrophobic residues in the gp120-gp41 putitative contact region, it is likely that drugs can be found that will bind to this region and may achieve the same effect as peptide binding. For instance a wide range of hydrophobic substances are known to block the action of calmodulin (compounds such as chlorpromazine and trifluorperazine bind to calmodulin in the presence of calcium and block its interaction with other proteins, Weiss & Levin, 1978), and therefore, a similar range of substances would be expected to disturb the gp120-gp41 interaction.

Broad classes of drugs which may be used in this invention would include the following:

1. Compounds with base functions to interact with the acid residues of the contact regions of gp120. These may include compounds with choline, amine, pyridine or guanidine functional groups.

2. Compounds with acidic functions to interact with the basic residues of the contact region of gp41. These may include compounds with carboxyl, sulphonate, sulphate or phosphate functional groups.

3. Compounds with hydrophobic features that bind to the hydrophobic regions contained in the contact region between gp41 and gp120. Such compounds may include phenothiazine, pimozide, haloperidol, chlordiazepoxide, phenylephrine, morphine, epinephrine, prostanoic acid, phospholipids, acylglycerols, digetoxigenin, benzamide, hydrolazine, phenoxyisobutyric acid, benzothiazides, steroid, probenecidsulfinpyrazone and trioxsalen.

The compounds according to the invention are useful for the treatment or prophylaxis of clinical conditions associated with HIV retroviral infection, for example, Kaposi's sarcoma, thrombocytopenic purpusa, AIDS-related complex, chronic neurological conditions and for patients carrying AIDS-antibodies or who are seropositive to the AIDS virus. Related compounds according to the invention are also useful for the treatment or prophylaxis of related viral infetions such as HIV-2 infections.

According to a further broad aspect of the invention, there is provided a method for the prevention or prophylaxis of viral infection, which comprises the administration to a subject of an effective amount of a compound, which is characterized by its ability to disrupt the interaction between viral envelope proteins which are crucial to viral uptake into target cells. With reference to the AIDS (HIV) virus, these compounds may be peptide or non-peptide compounds such as drugs, which disrupt the interaction between envelope proteins gp41 and gp120.

The compounds of the invention may be orally, topically or parenterally administered, and may be administered either alone, or in combination with one or more pharmaceutically acceptable carriers or experiments. Depending on the intended mode of administration, the compounds used may be administered in the form of solid, semi-solid or liquid dosage forms, such as, for example, tablets, pills, capsules, powders, liquids, suspensions, or the like, preferably in unit dosage forms suitable for single administration of precise dosages. Compositions containing the compounds of the invention will include a conventional pharmaceutical carrier or excipient, and, in addition, may include other medicinal agents, pharmaceutical agents, adjuvants, etc. Such excipients may include other proteins, such as, for example, human serum albumin or plasma preparations.

In general a suitable dose of the active compound will be in the range of 3.0 to 120 mg per kilogram body weight of the patient per day, preferably in the range of 6 to 90 mg per kilogram body weight per day and most preferably in the range 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1500 mg, preferably 20 to 1000 mg, and most preferably 50 to 700 mg of active ingredient per unit dosage form.

Preferably, a dose should be administered to achieve peak plasma concentrations of the active compound of from about 1 to 75 $\mu$M, preferably about 2 to 50 $\mu$M, most preferably about 3 to about 30 $\mu$M. The amounts here stated are not absolute, and will, of course, be dependent on the subject being treated, the severity of the afflication, the manner of administration and the judgement of a prescribing physician.

For solid compositions, conventional non-toxic solid carriers include, for example, pharmaceutical grades of mannitol, lactose, starch, or magnesium stearate.

Liquid pharmaceutically administrable compositions can, for example, be prepared by dissolving, dispersing, etc. the compounds of the invention and optional pharmaceutical adjuvants in a carrier, such as, for example, water, saline, aqueous dextrose, glycerol, ethanol, and the like, to thereby form a solution or suspension. If desired, the pharmaceutical composition to be adminitered may also contain minor amounts of nontoxic auxiliary substances such as wetting or emulsifying agents, pH buffering agents and the like, for example, sodium acetate or sorbitan monolaurate. Actual methods of preparing such dosage forms are known, or will be apparent, to those skilled in this art (for example, see Remington's Pharmaceutical Sciences, Mack Publishing Company, Easton, Pennsylvania, 15th Edition, 1975).

Individuals infected by the AIDS virus may be treated with the compounds and according to the methods of the invention over a prolonged period, which may cover the individuals remaining life span. Such treatment may prevent the spread of viral infection to uninfected cells. Moreover, viral DNA integrated into host cellular genetic material (i.e. in an inactive form) would be prevented from instigating the spread of viral particles upon activation at some later stage. Viral particles released from said activated cells could be rapidly rendered incapable of viral infection by disruption of gp41 and gp120 interaction.

Individuals likely to come into exposure with the AIDS virus, such as hemophiliacs, could be treated with the compounds and according to methods of the invention. Thus, any virus particles encountered by the individuals would be rapidly rendered non infectious.

Putative contact sequences for gp41 and gp120 are shown in detail in Table 1.

TABLE 1
--

The amino acid sequences of the envelope proteins are taken from Myer et al. (1988) for the isolate PV22, the isolate ROD for HIV2 and the isolate SIVMM142 for $SIV_{MAC}$. The matched charged residues are underlined and amino acid residue substitutions found in other isolates are given in parentheses. The single letter code for amino acids is as follows: A is alanine, C is cysteine, D is aspartic acid, E is glugamic acid, F is phenylalanine, G is glycine, H is histidine, I is isoleucine, K is lysine, L is leucine, M is methionine, N is asparagine, P is proline, Q is glutamine, R is arginine, S is serine, T is threonine, V is valine, W is tryptophan and Y is tyrosine.

HIU-1  gp41      U G I $\underline{K}^{574}$ Q L Q A $\underline{R}^{579}$ I L R V E $\underline{R}^{585}$ Y L K $\underline{D}^{589}$ Q Q
(571-591)                                    (V)                    (Q)
                                                                    (R)

HIU-1  gp120     D M V $\underline{E}^{102}$ Q M H E $\underline{D}^{107}$ I I S L W $\underline{D}^{113}$ Q S L $\underline{K}^{117}$ P C
(99-119)              (T)          (Q)
                      (N)          (I)
                      (K)

HIU-2  gp41      W G T $\underline{K}^{573}$ N L Q A $\underline{R}^{578}$ V T A I E $\underline{R}^{584}$ Y L Q $\underline{D}^{588}$ Q A
(570-590)

HIU-2 gp120      T V T $\underline{E}^{81}$ Q A I E $\underline{D}^{86}$ V W H L F $\underline{E}^{92}$ T S I $\underline{K}^{96}$ P C
(78-98)                               (V)          (N)

SIV$_{MAC}$      W G T $\underline{K}^{590}$ N L Q T R$^{595}$ U S A I E $\underline{K}^{601}$ Y L K $\underline{D}^{605}$ Q A
(587-607)                                      (T)                    (G)

SIV$_{MAC}$      T V T $\underline{E}^{84}$ Q A I E D$^{89}$ V W Q L F $\underline{E}^{95}$ T S I $\underline{K}^{99}$ P C
(81-101)

The contact sequence are characterized by a set of complimentary repeating charged residues (notably glutamic acid (E), aspartic acid (D), arginine (R) and lysine (K) interspersed with other amino acids. The arrangement of the four matched charged residues extending over 16 amino acids is conserved between the HIV 1 and HIV 2 strains. Spacing or the number of amino acids between each pair of complementary residues (4,5,3 residue spaces respectively) is conserved. This pattern is also observed for the closely related simian immunodificiency virus (see Table 1). Arrangements of the corresponding gp41 and gp120 contact sequences in the $\alpha$-helical configuration results in an amphipathic helix with correspondence between charge residue pairs and associated hydrophobic residues. The spacing does not correspond precisely to the 3.6 residues per turn of the $\alpha$-helix or the 3 residue per turn of the $3_{10}$ helix. Preliminary computer graphical analysis of the proposed contact arrangement indicates that the structures are highly complementary. The contact sequences identified herein are novel and specific, defining a single major contact sequence whose core is contained within a 16 residue pair of sequences and that as many as 3 out of 4 matching charge pair interactions can be accommodated.

The identification of the contact region described herein was surprising and depended on first knowing that a peptide corresponding to part of the gp120 sequence had antiviral activity (see below) and secondly asking the question if the antiviral sequence could associate with any other sequences found in the envelope proteins. Inspection of the gp41 sequence unexpectedly revealed a complimentary structure to the antiviral peptide sequence.

The contact sequence in gp41 (residues 571-591 for HIV 1) overlaps part of a sequence identified by Wang et al. (1986) and others (residues 579-601) that is major epitope and most viraemic patients have antibodies to this region.

The corresponding region in gp120 ($D^{99}$-$C^{118}$) contains a prominent T-cell epitope identified by Cease et al. (1987). The identification of this T-cell epitode was made with the aid of a computer algorithm that detected sites with a tendency to form amphipathic helices. Kaiser et al. (1984) have proposed a role for amphipathic helices in a variety of peptides and proteins including calcitonin. It is surprising that T and B cell epitopes would be associated with a contact region. Moreover, since epitopes are recognized by the immune surveillance system they are likely to be exposed and therefore corresponding synthetic peptides would not be expected to have antiviral activity unless they were derived from a region corresponding to the CD4 binding region. It is clear from the studies of Kowalski, et al. (1987) that the putative contact sequence in gp120 described herein is derived from regions distant to the CD4 binding region.

The strong B and T cell epitopes identified in gp41 and gp120 that overlap the contact regions described herein have been proposed as potentially important regions in the development of a vaccine. On the contrary, the identification of the contact regions made here suggests that in the intact virus particle these would be inaccessible. The masking of the B and T cell epitopes in the contact region of the infectious particle explains why the presence of circulating antibodies in patients fails to protect the viraemic patient from the progression of the disease. Since only the loss of gp120 from the infectious particle, which renders it non-infectious, results in an immune response to the major epitope in gp41, the resultant antibodies thus recognize non-infectious particles and therefore fail to provide protective immunity.

The invention will now be described, by way of example only, in the following non-limiting Examples.

## EXAMPLE 1:

### Peptide Synthesis:

Peptides can be prepared in a wide variety of ways including solution or solid phase strategies. Various manual, semi-automatic and automated synthesizers are commercially available and can be used in accordance with known protocols. The peptides cited herein were synthesized using the Merrifield procedure on an automated Applied Biosystems synthesizer (Hodges and Merrifield, 1975). The peptides were assembled on benzhydrylamine polystyrene/divinylbenzene resin. Benzyl side chain protection and BOC $\alpha$-amine protection were used. All residues were added by the direct dicyclohexylcarbodiimide method except for glutamine, asparagine, and arginine which were coupled as the hydroxybenzotriazote esters. The dinitrobenzene and formyl protective groups for histidine and tryptophan respectively were removed by treatment of the peptide resin amide with 20% (v/v) 2-mercaptoethanol, 1 M ethanolamine in dimethylformamide. The assembled peptide was cleaved from the resin and simultaneously deprotected by anhydrous HF (Stewart and Young, 1975). The free peptide was extracted in 60% (v/v) acetonitrile 0.1% (v/v) trifluoroacetic acid, rotary evaporated and lyophilized. Crude peptide was purified by reversed phase chromatography on a low pressure column (2.5 x 20 cm, Amicon $C_{18}$ reversed phase resin, 45-70 $\mu$ 250 A pore size) with a gradient of 0-60% acetonitrile. The purified peptide was lyophilized and characterized by high pressure liquid chromatography and amino acid analysis. For the extraction and purification of peptides suitable for therapeutic uses, non-toxic solvents are required, as would be apparent to those skilled in the art.

## EXAMPLE 2

### Antiviral Action of Contact Peptides:

This example demonstrates the antiviral activity of the following peptides:

```
gp120   (105-125)   H E D I I S L W D Q S L K P C V K L T P L
gp120   (105-117)   H E D I I S L W D Q S L K and
gp41    (572-591)   G I K Q L Q A R I L A V E R Y L K D Q Q.
```

Virus replication was assessed in peripheral blood lymphocyte cultures or peripheral blood lymphocytes, derived from a patent with acute lymphoblastic leukemia (CEM cells), by particle association reverse transcriptase (RT) activity and virus-associated syncytial formation (CPE). The HIV isolate used in all experiments was HTLV-IIIb.

CEM cells (4 x $10^5$/ml) were preincubated with synthetic peptide (200 $\mu$g/ml) in RPMI 640 medium containing 29.2 $\mu$g/ml glutamine, 100$\mu$g/ml streptomycin, 100 U/ml penicillin, 2mg/ml polybrene and 10% heat inactivated foetal calf serum for 1 hr at 37° in 5% $CO_2$. Following preincubation, HTLF IIIB H9 cell free supernatant was added at a multiplicity of infection of 0.0005 PFU/cell, according to the plaque assay using MT4 cells for 2 hr at 37° in 5% $CO_2$. The virus inoculum and peptide was removed with one wash in Eagles BME medium. CEM cells were resuspended in fresh medium containing peptide (100$\mu$g/ml) in a total volume of 2.0 ml. Cells were fed by exchanging 1.0 ml of cell-free supernatant fluid on days, 4, 7, 11 and 14 post-infection with fresh medium containing peptide (100$\mu$g/ml). Cell-free supernatant (500$\mu$l) was analyzed for reverse transcriptase activity. Positive controls without peptide and with a non-specific peptide corresponding to gizzard myosin light chain kinase (R R K W Q T G S A V) were included in this experiment as were negative controls containing no virus for cellular toxicity. Cell cultures were set up and assayed in quadruplicate. The synthetic peptides added did not exhibit any cytotoxicity as cell viability and numbers were equivalent to cultures without peptide.

The gp120 and gp41 test peptides showed significant viral inhibition, as measured by suppression of viral reverse transcriptase activity. Viral inhibition of over two orders of magnitude was observed. Control peptides did not effect viral activity.

REFERENCES:

Allan, J.S. et al., Science 229: 1091.
Barin, F. et al., Science 229: 1094 (1985).
Barre-Sinoussi, F. et al., Science 220: 868 (1983).
Blumenthal, et al., Proc. Natl. Acad, Sci., U.S.A., 82: 3187 (1985).
Cease, et al., Proc. Natl. Acad. Science, U.S.A., 84: 4249 (1987).
Cheong, et al., J. Biol. Chem. 261: 989 (1986).
Cox, et al., J. Biol. Chem. 260: 2527 (1985).
Dalgleish, A.G., et al., Nature (London) 312: 763 (1984).
Evans, J. Med. Chem. 30: 1229 (1987).
Gallo, R. et al., Science 224: 503 (1984).
Gaudreau et al., J. Biol. Chem. 262: 12413 (1987).
Hodges and Merrifield, Anal. Biochem. 65: 241 (1975).
Kaiser, E.T. and Ke'zdy, F.J., Science 223: 249 (1984).
Klatzman, D. et al., Nature (London) 312: 767 (1984).
Klevit et al., Biochemistry 24: 8152 (1985).
Kowalski, M. et al., Science 237: 1351 (1987).
Levy, J.A. et al., Science 225: 840 (1984).
Lifson, J.D. et al., Nature 323: 725 (1986).
Lucase et al., Biochemistry 25: 1458 (1986).
McClements et al., Virology 162: 270 (1988).
McDougal, J.S. et al., Science 231: 382 (1986a).
McDougal, J.S. et al., J. Immunol. 137: 2937-2944 (1986b).
McDougal, J.S. et al., J. Immunol. 135: 3151 (1985).
Montagnier, L. et al., Science 144: 283 (1983).
Myer, G. et al., Editors of Human retroviruses and AIDS, 1988 Data ase. Published by Theoretical Biology and Biophysics Los Alamos National Laboratory, Los Alamos, New Mexico.
Popovic, M., Gallo, R.C. and Mann, K.L., Clin. Res. 33: 560 (1984).
Popovic, M. et al., Science 220: 497 (1984).
Praise et al., J. Biol. Chem. 262: 12597 (1987).
Robey, W.G. et al., Science 229: 593 (1985).
Sarngadharan, M.D. et al., Science 220: 506 (1984).
Shaw, G. et al., Science 227: 177 (1985).
Sodroski, J. et al., Nature (London) 322: 470 (1986).
Stewart and Yong, Solid Phase Peptide Synthesis, pp. 44 and 66, W.H. Freeman, San Francisco (1975).
Veronese, F.D. et al., Science 229: 1402 (1985).
Wang, J.J.G. et al., Proc. Natl. Acad. Sci. U.S.A. 83: 6159 (1986).
Weiss and Levin, Adv. Cyclic Nucleotide Res. 99: 285 (1978).

**Claims**

1. Compounds for use in the treatment or prophylaxis of a Human Immunodeficiency Virus (HIV) infection, which upon contact with an HIV particle, are capable of preventing or disrupting interaction between the gp41 and gp120 envelope proteins of the said particle in the contact region between the said proteins.

2. Compounds according to claim 1 wherein said HIV infection is an HIV 1 infection.

3. Compounds according to claim 2 wherein prevention or disruption of interaction between said envelope proteins is affected in the contact region defined by the following amino acid sequences:

```
            571      574            579              585          589  591
gp41    ...W   G  I  K   Q  L  Q  A  R   I  L  A  V  E  R   Y  L  K   D   Q Q...
            99       102            107              113          117
gp120   ...D   M  V  E   Q  M  H  E  D   I  I  S  L  W  D   Q  S  L   K   P C...
```

4. Compounds according to claim 1 wherein said HIV Infection is an HIV II infection.

5. Compounds according to claim 4 wherein prevention or disruption of interaction between said envelope proteins is affected in the contact region defined by the following amino acid sequence:

```
            571      574            579              585          589  591
gp41    ...W   G  T  K   N  L  Q  A  R   V  T  A  I  E  K   Y  L  Q   D   Q A...
            78       81             86               92           96   98
gp120   ...T   V  T  E   Q  A  I  E  D.  V  W  H  L  F  E   T  S  I   K   P C...
```

6. Compounds according to claims 3 or 5 which mimic at least a portion of the said amino acid sequences of either the gp41 or gp120 protein.

7. Compounds according to claim 6 containing the following amino acid sequence:
H E D I I S L W D Q S L K P C V K L T P L
or
G I K Q L Q A R I L A V E R Y K D Q Q
or parts thereof.

8. Compounds according to claim 7 containing the following amino acid sequence:
H E D I I S L W D Q S L K.

9. Compounds according to claim 1 containing basic functions capable of interacting with the acidic residues of the gp120 protein in the said contact region.

10. Compounds according to claim 1 containing acidic functions capable of interacting with the basic residues of the contact region of the gp41 protein in the said contact region.

11. Compounds according to claim 1 containing hydrophobic functions capable of interacting with hydrophobic regions of the gp41 and or gp120 proteins in the said contact region.

12. Compounds according to claim 1 for use in the treatment or prophylaxis of Acquired Immune Deficiency Syndrome (AIDS) or AIDS Related Complex (ARC).

13. Compounds according to claim 1 for use in the treatment or prophylaxis of HIV-positive individuals.

14. Use of a compounds as defined in claim 1 in the manufacture of a medicament for the treatment or prophylaxis of an HIV infection.

15. Peptide compounds having the amino acid sequence:
H E D I I S L W D Q S L K P C V K L T P L
or
G I K Q L Q A R I L A V E R Y L K D Q Q
or parts thereof.

16. Peptide compounds having the following amino acid sequence:
H E D I I S L W D Q S L K.

17. Pharmaceutical formulations comprising a compound as defined in claim 1 together with at least one pharmaceutically acceptable carrier or excipient.

18. Pharmaceutical formulations comprising a peptide compound as defined in claims 15 or 16 together with at least one pharmaceutically acceptable carrier or excipient therefor.

19. A process for the preparation of a peptide compound as defined in claims 15 or 16, or any compound as defined in any one of claims 1 to 13, comprising processes known per se in the art of peptide synthesis.